# EUROPEAN PATENT SPECIFICATION

(11) **EP 0 573 341 B1**
(45) Date of publication and mention of the grant of the patent: **01.07.1998**
(21) Application number: 93401379.8
(22) Date of filing: 28.05.1993
(51) Int. Cl.: C11D 1/94, C11D 17/00, A61K 7/08, A61K 7/50

(54) **High foaming nonionic surfactant based liquid detergent**
Flüssiges Reinigungsmittel auf der Basis von starkschäumenden, nichtionischen, oberflächenaktiven Mitteln
Composition détergente liquide à base de surfactant non ionique très moussant

(30) Priority: 03.06.1992 US 893138
(43) Date of publication of application: 08.12.1993
(73) Proprietor: Colgate-Palmolive Company, New York, N.Y. 10022-7499 (US)
(72) Inventor: Repinec, Stephen T., Jr., Flemington, New Jersey (US); Gomes, Gilbert S., Somerset, New Jersey (US); Erilli, Rita, B-4000 Rocourt (BE)
(74) Representative: Le Guen, Gérard

(56) References cited:
- EP-A- 0 155 737
- EP-A- 0 208 440
- WO-A-91/00138
- US-A- 3 950 417
- US-A- 4 595 526
- US-A- 4 992 107

## Description

### BACKGROUND OF THE INVENTION

The present invention relates to novel light duty liquid detergent compositions with high foaming properties, containing a nonionic surfactant as the major active ingredient supplemented with lesser amounts of a specific group of anionic surfactants and even smaller amounts of a Zwitterionic betaine surfactant in an aqueous medium.

Nonionic surfactants are in general chemically inert and stable toward pH change and are therefore well suited for mixing and formulation with other materials. The superior performance of nonionic surfactants on the removal of oily soil is well recognized. Nonionic surfactants are also known to be mild to human skin. However, as a class, nonionic surfactants are known to be low or moderate foamers. Consequently, for detergents which require copious and stable foam, the application of nonionic surfactants is limited. There have been substantial interest and efforts to develop a high foaming detergent with nonionic surfactants as the major ingredient. Yet, little has been achieved.

The prior art is replete with light duty liquid detergent compositions containing nonionic surfactants in combination with anionic and/or betaine surfactants wherein the nonionic detergent is not the major active surfactant, as shown in U.S. Patent No. 3,658,985 wherein an anionic based shampoo contains a minor amount of a fatty acid alkanolamide. U.S. Patent No. 3,769,398 discloses a betaine-based shampoo containing minor amounts of nonionic surfactants. This patent states that the low foaming properties of nonionic detergents renders its use in shampoo compositions non-preferred. U.S. Patent No. 4,329,335 also discloses a shampoo containing a betaine surfactant as the major ingredient and minor amounts of a nonionic surfactant and of a fatty acid mono- or diethanolamide. U.S. Patent No. 4,259,204 discloses a shampoo comprising 0.8-20% by weight of an anionic phosphoric acid ester and one additional surfactant which may be either anionic, amphoteric, or nonionic. U.S. Patent No. 4,329,334 discloses an anionic-amphoteric based shampoo containing a major amount of anionic surfactant and lesser amounts of a betaine and nonionic surfactants.

U.S. Patent No. 3,935,129 discloses a liquid cleaning composition based on the alkali metal silicate content and containing five basic ingredients, namely, urea, glycerin, triethanolamine, an anionic detergent and a nonionic detergent. The silicate content determines the amount of anionic and/or nonionic detergent in the liquid cleaning composition. However, the foaming property of these detergent compositions is not discussed therein.

U.S. Patent No. 4,129,515 discloses a heavy duty liquid detergent for laundering fabrics comprising a mixture of substantially equal amounts of anionic and nonionic surfactants alkanolamines and magnesium salts, and, optionally, zwitterionic surfactants as suds modifiers.

U.S. Patent No. 4,224,195 discloses an aqueous detergent composition for laundering socks or stockings comprising a specific group of nonionic detergents, namely, an ethylene oxide of a secondary alcohol, a specific group of anionic detergents, namely, a sulfuric ester salt of an ethylene oxide adduct of a secondary alcohol, and an amphoteric surfactant which may be a betaine, wherein either the anionic or nonionic surfactant may be the major ingredient. The specific class of anionics utilized in this patent is the very same group of anionic detergents expressly excluded in present invention in order to eliminate the alkanol ethoxylate sulfation process and the potential dioxane toxicity problem. Furthermore, this patent finds heavily foaming detergents undesirable for the purpose of washing socks.

The prior art also discloses detergent compositions containing all nonionic surfactants as shown in U.S. Patent Nos. 4,154,706 and 4,329,336 wherein the shampoo compositions contain a plurality of particular nonionic surfactants in order to effect desirable foaming and detersive properties despite the fact that nonionic surfactants are usually deficient in such properties.

U.S. Patent No. 4,013,787 discloses a piperazine based polymer in conditioning and shampoo compositions which may contain all nonionic surfactant or all anionic surfactant.

U.S. Patent No. 4,450,091 discloses high viscosity shampoo compositions containing a blend of an amphoteric betaine surfactant, a polyoxybutylenepolyoxyethylene nonionic detergent, an anionic surfactant, a fatty acid alkanolamide and a polyoxyalkylene glycol fatty ester. But, none of the exemplified compositions contains an active ingredient mixture wherein the nonionic detergent is present in major proportion, probably due to the low foaming properties of the polyoxybutylene polyoxyethylene nonionic detergent.

U.S. Patent No. 4,595,526 describes a composition comprising a nonionic surfactant, betaine surfactant, an anionic surfacant and a C₁₂-C₁₄ fatty acid monethanolamide foam stabilizer.

However, none of the above-cited patents discloses a high foaming, nonionic based, liquid detergent composition containing a nonionic surfactant as a major active ingredient and minor amounts of a supplementary high foaming anionic sulfate or sulfonate surfactant excluding ethoxylated alcohol ether sulfates, a supplementary foaming zwitterionic surfactant selected from betaine type surfactants as the three essential ingredients, wherein the nonionic ingredient constitutes more than 50% of the total surfactant content and the composition does not contain any amine oxide, amide type compound.

### SUMMARY OF THE INVENTION

It has now been found that a high foaming liquid detergent can be formulated with a nonionic surfactant as the major active ingredient which has desirable cleaning properties, mildness to the human skin and avoids the dioxane toxicity problem associated with the sulfation process of manufacturing anionic ethoxylated alcohol ether sulfates.

Accordingly one object of the invention is to provide novel, high foaming, nonionic based, light duty liquid detergent compositions containing a nonionic ionic surfactant at a concentration of at least 50% of the total surfactant content.

Another object of this invention is to provide novel, nonionic based, liquid detergent compositions containing a major amount of nonionic surfactant supplemented with lesser amounts of an anionic surfactant, and a zwitterionic betaine surfactant wherein the composition does not contain amine oxide, a fatty acid alkanolamide foam stabilizer.

Still another object of this invention is to provide a novel, nonionic based, liquid detergent with desirable high foaming and cleaning properties which is mild to the human skin.

A further object of this invention is to provide a novel, nonionic based liquid detergent containing a supplemental anionic surfactant excluding the ethoxylated alkyl ether sulfates which eliminates the alkanol ethoxylate sulfation process and the potential dioxane toxicity problem.

Additional objects, advantages and novel features of the invention will be set forth in part in the description which follows, and in part will become apparent to those skilled in the art upon examination of the following or may be learned by practice of the invention. The objects and advantages of the invention may be realized and attained by means of the instrumentalities and combinations particularly pointed out in the appended claims.

To achieve the foregoing and other objects and in accordance with the purpose of the present invention, as embodied and broadly described herein the novel, high foaming, nonionic based, light duty liquid detergent of this invention comprises three essential surfactants a water soluble, ethoxylated, nonionic surfactant as the major active ingredient in an amount exceeding 50% by weight of the total surfactant content; a supplemental amount of a foaming anionic surfactant selected from the group consisting of water soluble organic sulfates and organic sulfonates, excluding the ethoxylated alkyl ether sulfates; a lesser amount of a foaming water soluble, and a zwitterionic surfactant selected from the class of betaines dissolved in an aqueous vehicle, wherein the composition does not contain any amine oxide, amide or alkanolamide ingredients.

More specifically, the present invention relates to a high foaming, nonionic based, liquid detergent containing more than 50% by weight of the total surfactant content of a nonionic surfactant selected from the group consisting of water soluble primary aliphatic alcohol ethoxylates secondary aliphatic alcohol ethoxylates, alkyl phenol ethoxylates and alcohol ethylene oxide propylene oxide condensates; and supplementary amounts of an anionic surfactant selected from the group consisting of water soluble salts of C₈-C₁₈ alkyl sulfates, C₈-C₁₆ benzene sulfonates, C₁₀-C₂₀ paraffin sulfonates, alpha C₁₀-C₂₄ olefin sulfonates, C₈-C₁₈ alkyl suloacetates, C₈-C₁₈ alkyl sulfosuccinate esters, C₈-C₁₈ acyl isethionates and C₈-C₁₈ acyl taurates; and a water soluble zwitterionic betaine surfactant; the total content of said supplementary surfactants, constituting less that 50% by weight of the total surfactant content, dissolved in an aqueous vehicle.

This particular combination of three ingredients in the proportions, by weight, of more than 50% of the nonionic surfactant of the total surfactant content and 15 to 48% of the sum of anionic surfactant and betaine surfactant, is critical to the high foaming and desirable cleansing properties of present liquid detergent and the retention of the mildness to the skin property. The total amount of surfactants may constitute 10%-55%, preferably 20%-40%, most preferably 25%-35%, by weight of the liquid composition.

### DETAILED DESCRIPTION OF THE INVENTION

The nonionic surfactant which constitutes the major ingredient in present liquid detergent is present in amounts of about 10%-30%, preferably 13%-25%, most preferably 16%-22%, by weight of the composition and provides superior performance in the removal of oily soil and mildness to human skin.

The water soluble nonionic surfactants utilized in this invention are commercially well known and include the primary aliphatic alcohol ethoxylates, secondary aliphatic alcohol ethoxylates, alkylphenol ethoxylates and ethylene-oxide-propylene oxide condensates on primary alkanols, such a Plurafacs® (BASF) and condensates of ethylene oxide with sorbitan fatty acid esters such as the Tweens® (ICI). The nonionic synthetic organic detergents generally are the condensation products of an organic aliphatic or alkyl aromatic hydrophobic compound and hydrophilic ethylene oxide groups. Practically any hydrophobic compound having a carboxy, hydroxy, amido, or amino group with a free hydrogen attached to the nitrogen can be condensed with ethylene oxide or with the polyhydration product thereof, polyethylene glycol, to form a water-soluble nonionic detergent. Further, the length of the polyethenoxy chain can be adjusted to achieve the desired balance between the hydrophobic and hydrophilic elements.

The nonionic detergent class includes the condensation products of a higher alcohol (e.g., an alkanol containing about 8 to 18 carbon atoms in a straight or branched chain configuration) condensed with about 5 to 30 moles of ethylene oxide, for example, laurylmyristyl alcohol condensed with about 16 moles of ethylene oxide (EO), tridecanol condensed with about 6 to moles of EO, myristyl alcohol condensed with about 10 moles of EO per mole of myristyl alcohol, the condensation product of EO with a hear-cut of coconut fatty alcohol containing a mixture of fatty alcohols with alkyl chains varying from 10 to about 14 carbon atoms in length and wherein the condensate contains either about 6 moles of EO per mole of total alcohol or about 9 moles of EO per mole of alcohol and tallow alcohol ethoxylates containing 6 EO to 11 EO per mole of alcohol.

A preferred group of the foregoing nonionic surfactants are the Neodol ethoxylates® (Shell Co.), which are higher aliphatic, primary alcohol containing about 9-15 carbon atoms, such as C₉-C₁₁ alkanol condensed with 8 moles of ethylene oxide (Neodol® 91-8), C₁₂₋₁₅ alkanol condensed with 6,5 moles ethylene oxide (Neodol® 23-6.5), C₁₂₋₁₅ alkanol condensed with 12 moles ethylene oxide (Neodol® 25-12), C₁₄₋₁₅ alkanol condensed with 13 moles ethylene oxide (Neodol® 45-13), and the like. Such ethoxamers have an HLB (hydrophobic lipophilic balance) value of about 8-15 and give good/W emulsification, whereas ethoxamers with HLB values below 8 contain less than 5 ethyleneoxy groups and tend to be poor emulsifiers and poor detergents.

Additional satisfactory water soluble alcohol ethylene oxide condensates are the condensation products of a secondary aliphatic alcohol containing 8 to 18 carbon atoms in a straight or branched chain configuration condensed with 5 to 30 moles of ethylene oxide. Examples of commercially available nonionic detergents of the foregoing type are C₁₁-C₁₅ secondary alkanol condensed with either 9 EO (Tergitol® 15-S-9) or 12 EO (Tergitol® 15-S-12) marketed by Union Carbide.

Other suitable nonionic detergents include the polyethylene oxide condensates of one mole of alkyl phenol containing from 8 to 18 carbon atoms in a straight- or branched chain alkyl group with 5 to 30 moles of ethylene oxide. Specific examples of alkyl phenol ethoxylates include nonyl condensed with about 9.5 moles of EO per mole of nonyl phenol, dinonyl phenol condensed with about 12 moles of EO per mole of phenol, dinonyl phenol condensed with about 15 moles of EO per mole of phenol and di-isoctylphenol condensed with about 15 moles of EO per mole of phenol. Commercially available nonionic surfactants of this type include Igepal® CO-630 (nonyl phenol ethoxylate) marketed by GAF Corporation.

Also among the satisfactory nonionic detergents are the water-soluble condensation products of a C₈-C₂₀ alkanoi with a heteric mixture of ethylene oxide and propylene oxide wherein the weight ratio of ethylene oxide to propylene oxide is from 2.5:1 to 4:1, preferably 2.8:1-3.3:1, with the total of the ethylene oxide and propylene oxide (including the terminal ethanol or propanol group) being from 60-85%, preferably 70-80%, by weight. Such detergents are commercially available from BASF-Wyandotte and a particularly preferred detergent is a C₁₀-C₁₆ alkanol condensate with ethylene oxide and propylene oxide, the weight ratio of ethylene oxide to propylene oxide being 3:1 and the total alkoxy content being about 75% by weight.

Condensates of 2 to 30 moles of ethylene oxide with sorbitan mono- and tri-C₁₀-C₂₀ alkanoic acid esters having a HLB of 8 to 15 also may be employed as the nonionic detergent ingredient in the described shampoo. These surfactants are well known and are available from Imperial Chemical Industries under the Tween® trade name. Suitable surfactants include polyoxyethylene (4) sorbitan monolaurate, polyoxyethylene (4) sorbitan monostearate, polyoxyethylene (20) sorbitan trioleate and polyoxyethylene (20) sorbitan tristearate.

Other suitable water-soluble nonionic detergents which are less preferred are marketed under the trade name "Pluronics®." The compounds are formed by condensing ethylene oxide with a hydrophobic base formed by the condensation of propylene oxide with propylene glycol. The molecular weight of the hydrophobic portion of the molecule is of the order of 950 to 4000 and preferably 200 to 2,500. The addition of polyoxyethylene radicals to the hydrophobic portion tends to increase the solubility of the molecule as a whole so as to make the surfactant water-soluble. The molecular weight of the block polymers varies from 1,000 to 15,000 and the polyethylene oxide content may comprise 20% to 80% by weight. Preferably, these surfactants will be in liquid form and satisfactory surfactants are available as grades L 62 and L 64.

The anionic surfactant, which is an essential ingredient of present liquid detergent composition, constitutes about 1% to 10%, preferably 2%-9%, most preferably 3%-8%, by weight thereof and provides good foaming properties. However, preferably reduced amounts are utilized in order to enhance the mildness of the skin property desired in the inventive compositions, and thus, the weight ratio of nonionic detergent to anionic should exceed about 3:1. In addition, the particular group of anionic surfactants utilized excludes the C₈-C₁₈ alkyl polyethenoxy ether sulfate surfactants in order to avoid the dioxane toxicity associated with the process of sulfation of ethoxylated alcohols. Thus, the ethoxylated alcohol ether sulfates are expressly excluded from the specific group of anionic surfactants utilized.

The anionic surfactants which may be used in the nonionic based liquid detergent of this invention are water soluble such as triethanolamine and include the sodium, potassium, ammonium and ethanolammonium salts of C₈-C₁₈ alkyl sulfates such as lauryl sulfate, myristyl sulfate and the like except for triethanolamine lauryl sulfate; linear C₈-C₁₆ alkyl benzene sulfonates; C₁₀-C₂₀ paraffin sulfonates; alpha olefin sulfonates containing 10-24 carbon atoms; C₈-C₁₈ alkyl sulfoacetates; C₈-C₁₈ alkyl sulfosuccinate esters; C₈-C₁₈ acyl isethionates; and C₈C-₁₈ acyl taurates. Preferred anionic surfactants are the water soluble C₁₂-C₁₆ alkyl sulfates, the C₁₀-C₁₅ alkylbenzene sulfonates, the C₁₃-C₁₇ paraffin sulfonates and the alpha C₁₂-C₁₈ olefin sulfonates.

The water-soluble zwitterionic surfactant, which is also an essential ingredient of present liquid detergent composition, constitutes 0.5-10%, preferably 2%-9%, most preferably 7%-8%, by weight and provides good foaming properties and mildness to the present nonionic based liquid detergent. The zwitterionic surfactant is a water soluble betaine having the general formula: 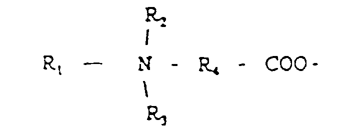 wherein R₁ is an alkyl group having 10 to about 20 carbon atoms, preferably 12 to 16 carbon atoms, or the amido radical: 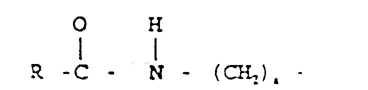 wherein R is an alkyl group having 9 to 19 carbon atoms and a is the integer 1 to 4; R₂ and R₃ are each alkyl groups having 1 to 3 carbons and preferably 1 carbon: R₄ is an alkylene or hydroxyalkylene group having from 1 to 4 carbon atoms and, optionally, one hydroxyl group. Typical alkyldimethyl betaines include decyl dimethyl betaine or 2-(N-decyl-N, N-dimethyl-ammonia) acetate, coco dimethyl betaine or 2-(N-coco N, N-dimethylammonio) acetate, myristyl dimethyl betaine, palmityl dimethyl betaine, lauryl diemthyl betaine, cetyl dimethyl betaine, stearyl dimethyl betaine, etc. The amidobetaines similarly include cocoamidoethylbetaine, cocoamidopropyl betaine and the like. A preferred betaine is coco (C₈-C₁₈) amidopropyl dimethyl betaine.

All of the aforesaid three ingredients in this light duty liquid detergent are water soluble or water dispersible and remain so during storage.

This particular combination of anionic surfactant and betaine surfactant, provides a detergent system which coacts with the nonionic surfactant to product a liquid detergent composition with desirable foaming, foam stability, detersive properties and mildness to human skin. Surprisingly, the resultant homogeneous liquid detergent exhibits the same or better foam performance, both as to initial foam volume and stability of foam in the presence of soils, and cleaning efficacy as an anionic based light duty liquid detergent (LDLD) as shown in the following Examples.

The essential ingredients discussed above are solubilized in an aqueous medium comprising water and optionally, solubilizing ingredients such as C₂-C₃ mono and di-hydroxy alkanols, e.g., ethanol, isopropanol and propylene glycol. Suitable water soluble hydrotropic salts include sodium, potassium, ammonium and mono-, di- and triethanolammonium salts. While the aqueous medium is primarily water, preferably said solubilizing agents are included in order to control the viscosity of the liquid composition and to control low temperature cloud clear properties. Usually, it is desirable to maintain clarity to a temperature in the range of 5°C to 10°C. Therefore, the proportion of solubilizer generally will be from 1%-15%, preferably 2%-12%, most preferably 3%-8%, by weight of the detergent composition with the proportion of ethanol, when present, being 5% of weight or less in order to provide a composition having a flash point above about 46°C. Preferably the solubilizing ingredient will be a mixture of ethanol and either sodium xylene sulfonate or sodium cumene sulfonate or a mixture of said sulfonates.

The foregoing solubilizing ingredients also facilitate the manufacture of the inventive compositions because they tend to inhibit gel formation.

In addition to the previously mentioned essential and optional constituents of the light duty liquid detergent, one may also employ normal and conventional adjuvants, provided they do not adversely affect the properties of the detergent. Thus, there may be used various coloring agents and perfumes; ultraviolet light absorbers such as the Uvinuls®, which are products of GAF Corporation; sequestering agents such as ethylene diamine tetraacetates; magnesium sulfate heptahydrate; pearlescing agents and opacifiers; pH modifiers; etc. The proportion of such adjuvant materials, in total will normally not exceed 15% of weight of the detergent composition, and the percentages of most of such individual components will be a maximum of 5% by weigh and preferably less than about 2% by weight. Sodium formate can be included in the formula as a perservative at a concentration of 0.1 to 40%. Sodium bisulfite can be used as a color stabilizer at a concentration of 0.01 to 0.2 wt.%

The present nonionic based light duty liquid detergents such as dishwashing liquids are readily made by simple mixing methods from readily available components which, on storage, do not adversely affect the entire composition. However, it is preferred that the nonionic surfactant be mixed with the solubilizing ingredients, e.g., ethanol and, if present, prior to the addition of the water to prevent possible gelation. The nonionic based surfactant system is prepared by sequentially adding with agitation the anionic surfactant and the betaine to the non-ionic surfactant which has been previously mixed with a solubilizing agent such as ethyl alcohol and/or sodium xylene sulfonate to assist in solubilizing said surfactants, and then adding with agitation the formula amount of water to form an aqueous solution of the nonionic based surfactant system. The use of mild heating (up to 100°C.) assists in the solubilization of the surfactants. The viscosities are adjustable by changing the total percentage of active ingredients. Usually, no thickening agent is added, but thickeners may be added if higher viscosity liquids are desired. In all such cases the product made will be pourable from a relatively narrow mouth bottle (1.5 cm. diameter) or opening, and the viscosity of the detergent formulation will not be so low as to be like water. The viscosity of the detergent desirably will be at least 100 centipoises mPa.s (cps) at room temperature, but may be up to about 1,000 mPa.s (centipoises) as measured with a Brookfield Viscometer using a number 3 spindle rotating at 12 rpm. Its viscosity may approximate those of commercially acceptable detergents now on the market. The detergent viscosity and the detergent itself remain stable on storage for lengthy periods of time, without color changes or settling out of any insoluble materials. The pH of this formation is substantially neutral to skin, e.g., 4.5 to 8 and preferably about 5.5.

These products have unexpectedly desirably properties. For example, the foam quality and detersive property is equal to or better than standard light duty liquid detergents while using a nonionic surfactant as the primary surfactant and minimal amounts of anionic surfactant, thereby achieving a mild, non-irritating liquid detergent.

The following examples are merely illustrative of the invention and are not to be construed as limiting thereof.

### EXAMPLE 1

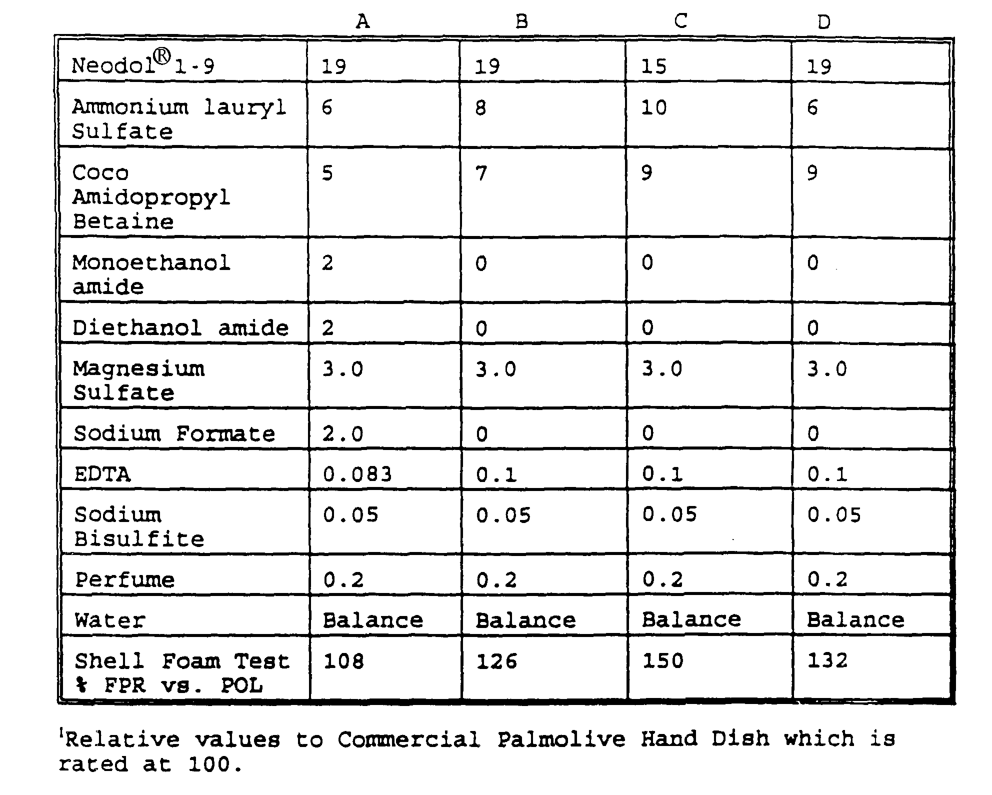

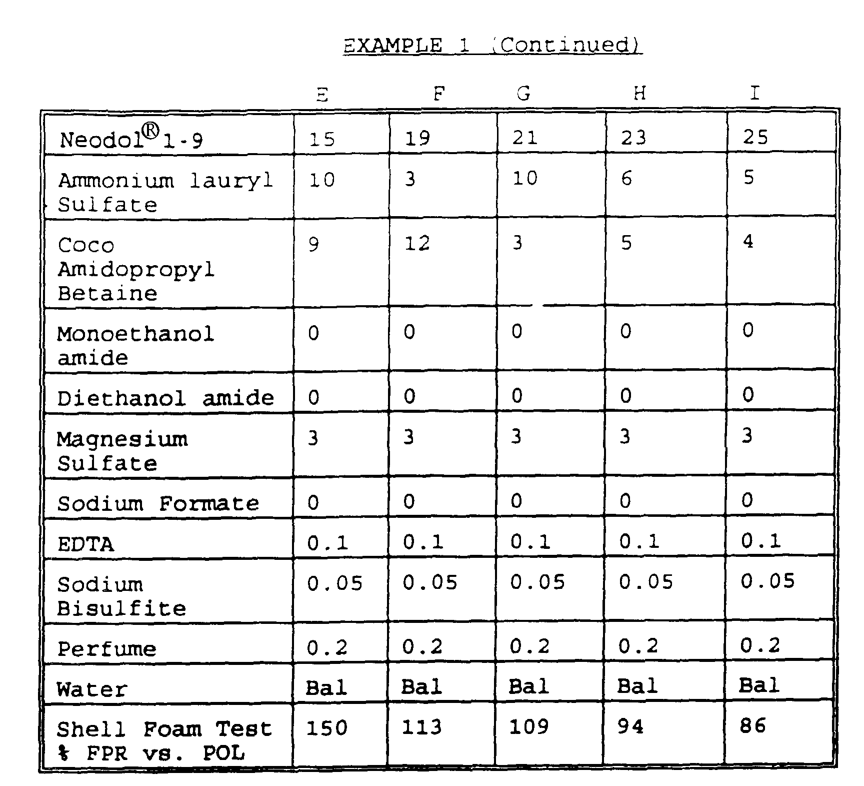

### EXAMPLE 2

Formula A of Example 1 (composition of U. S. Patent 4,595,526) and Formula B were compared in a Hand Dish Wash Test. The test was run with both Ragu Spaghetti Sauce and Shell Soil. The results show that dish washing performance between the U. S. formula and the European formula is similar. The test was run blind with each trial consisting of three runs of each product and was done in both 300 and 150 ppm water hardness.

Results of Formula A Versus U. S. using Ragu

| Trial | Sample | #Plates (300ppm) | #Plates(150ppm) |
|---|---|---|---|
| 1 | A | 29 | 27 |
| | B | 23 | 27 |
| | | | |
| 2 | A | 31 | 26 |
| | B | 26 | 27 |
| | | | |
| 3 | A | 31 | 28 |
| | B | 29 | 27 |
| | | | |
| Average | | | |
| | | | |
| | A | 30.3 | 27 |
| | B | 26 | 27 |

Results of Formula A Versus B using Shell Soil

| Trial | Sample | #Plates (300ppm) | #Plates(150ppm) |
|---|---|---|---|
| 1 | A | 14 | 14 |
| | B | 13 | 13 |
| | | | |
| 2 | A | 14 | 14 |
| | B | 13 | 12 |
| | | | |
| 3 | A | 14 | 13 |
| | B | 13 | 12 |
| | | | |
| Average | | | |
| | | | |
| | A | 14 | 13.7 |
| | B | 13 | 12.3 |

## Claims

1. A high foaming, nonionic surfactant-based, light duty, liquid detergent comprising approximately, by weight,
(a) 10% to 30% of a water soluble nonionic surfactant selected from the group consisting of primary and secondary C₈-C₁₈ alkanol condensates with 5 to 30 moles of ethylene oxide, condensates of C₈-C₁₈ alkylphenol with 5 to 30 moles of ethylene oxide, condensates of C₈-C₂₀ alkanol with a heteric mixture of ethylene oxide and propylene oxide having a weight ratio of ethylene oxide to propylene oxide from 2.5:1 to 4:1 and a total alkylene oxide content of 60% to 85% by weight and condensates of 2 to 30 moles of ethylene oxide with sorbitan mono and tri- C₁₀-C₂₀ alkanoic acid esters having an HLB of 8 to 15;
(b) 1% to 10% of a water-soluble anionic detergent selected from the group consisting of C₈-C₁₈ alkyl sulfates, C₈-C₁₆ alkylbenzene sulfonates, C₁₀-C₂₀ paraffin sulfonates, C₁₀-C₂₄ alpha olefin sulfonates and C₈-C₁₈ alkyl sulfosuccinate esters, C₈-C₁₈ acyl isethionates and C₈-C₁₈ acyl taurates; and
(c) 0.5% to 10% of a water-soluble betaine; and
(d) balance being water as an aqueous medium in which said nononic surfactant, said anionic detergent and said betaine are solubilized in said water, wherein the sum of C and B being from 15% to 48% by weight of the composition of the total surfactant content, said nonionic surfactant being in excess of 50% by weight of the total surfactant content and said composition being free of anionic alkyl ether polyethenoxy sulfate detergent, anionic triethanolamine lauryl sulfate detergent, amides, amine oxides, and alkanolamides.

2. A liquid detergent composition according to claim 1 which includes, in addition, 1% to 15% by weight of a solubilizing agent selected from the group consisting of C₂-C₃ mono- and di-hydroxy alkanols, water soluble salts of C₁-C₃ substituted benzene sulfonate hydrotropes and mixtures thereof.

3. A liquid detergent composition according to claim 2 wherein ethanol is present in the amount of 5% by weight or less.

4. A liquid detergent composition according to claim 2 wherein said nonionic surfactant is said condensate of a primary C₈-C₁₈ alkanol with 5-30 moles of ethylene oxide.

5. A liquid detergent composition according to claim 4 wherein said anionic detergent is selected from the group consisting of C₁₂-C₁₆ alkyl sulfates, C₁₀-C₁₅ alkylbenzene sulfonates, C₁₃-C₁₇ paraffin sulfonates and C₁₂-C₁₈ alpha olefin sulfonates.

6. A liquid detergent composition according to claim 1 wherein said nonionic surfactant is present in an amount of 16% to 22% by weight, said anionic detergent is present in an amount of 2% to 9% by weight and said betaine is present in an amount of 2% to 9% by weight.

7. A liquid detergent composition according to claim 6 wherein said anionic detergent is a C₁₂-C₁₆ alkyl sulfate.

8. A liquid detergent composition according to Claim 1 further including a preservative.

9. A liquid detergent composition according to Claim 1 further including a color stabilizer.

10. A method of preparing the liquid detergent of claim 2 which comprises the steps of first mixing said nonionic surfactant with the solubilizing agent, sequentially adding with agitation said anionic surfactant and said betaine and lastly adding with agitation, the formula amount of water to form an aqueous solution of the nonionic based surfactant composition.

## Patentansprüche

1. Stark schäumendes, auf nichtionischem Tensid basierendes, flüssiges Feinreinigungsmittel, das, bezogen auf das Gewicht, ungefähr
(a) 10 bis 30 % in Wasser lösliches nichtionisches Tensid ausgewählt aus der Gruppe bestehend aus primären und sekundären C₈- bis C₁₈-Alkanolkondensaten mit 5 bis 30 Molen Ethylenoxid, Kondensaten von C₈- bis C₁₈-Alkylphenol mit 5 bis 30 Molen Ethylenoxid, Kondensaten von C₈- bis C₂₀-Alkanol mit einer heterischen Mischung aus Ethylenoxid und Propylenoxid mit einem Gewichtsverhätnis von Ethylenoxid : Propylenoxid von 2,5 : 1 bis 4 : 1 und einem Gesamtalkylenoxidgehalt von 60 bis 85 Gew.-% und Kondensaten von 2 bis 30 Molen Ethylenoxid mit Sorbitanmono- und -tri-C₁₀- bis C₂₀-alkansäureestern mit HLB-Werten von 8 bis 15,
(b) 1 bis 10 % in Wasser lösliches anionisches Reinigungsmittel ausgewählt aus der Gruppe bestehend aus C₈- bis C₁₈-Alkylsulfaten, C₈- bis C₁₆-Alkylbenzolsulfonaten, C₁₀- bis C₂₀-Paraffinsulfonaten, C₁₀ bis C₂₄-α-Olefinsulfonaten und C₈- bis C₁₈-Alkylsulfosuccinatestern, C₈- bis C₁₈-Acylisethionaten und C₈- bis C₁₈-Acyltauraten, und
(c) 0,5 bis 10 % in Wasser lösliches Betain umfaßt, wobei
(d) der Rest Wasser als wäßriges Medium ist, in dem das nichtionische Tensid, das anionische Reinigungsmittel und das Betain solubilisiert sind, die Summe von c) und b) 15 bis 48 Gew.-% der Zusammensetzung des gesamten Tensidgehalts ist, das nichtionische Tensid mehr als 50 Gew.-% des gesamten Tensidgehalts ausmacht und die Zusammensetzung frei von anionischem Alkyletherpolyethenoxysulfat-Reinigungsmittel, anionischem Triethanolaminlaurylsulfat-Reinigungsmittel, Amiden, Aminoxiden und Alkanolamiden ist.

2. Flüssige Reinigungsmittelzusammensetzung nach Anspruch 1, die zusätzlich 1 bis 15 Gew.-% Solubilisierungsmittel ausgewählt aus der Gruppe bestehend aus C₂- bis C₃-Mono- und Dihydroxyalkanolen, in Wasser löslichen Salzen von C₁ bis C₃- substituierten Benzolsulfonat-Hydrotropen und Mischungen derselben enthält.

3. Flüssige Reinigungsmittelzusammensetzung nach Anspruch 2, bei der Ethanol in einer Menge von 5 Gew.-% oder weniger vorhanden ist.

4. Flüssige Reinigungsmittelzusammensetzung nach Anspruch 2, bei der das nichtionische Tensid das Kondensat eines primären C₈- bis C₁₈-Alkanols mit 5 bis 30 Molen Ethylenoxid ist.

5. Flüssige Reinigungsmittelzusammensetzung nach Anspruch 4, bei der das anionische Reinigungsmittel ausgewählt ist aus der Gruppe bestehend aus C₁₂- bis C₁₆-Alkylsulfaten, C₁₀- bis C₁₅-Alkylbenzolsulfonaten, C₁₃- bis C₁₇-Paraffinsulfonaten und C₁₂- bis C₁₈-α-Olefinsulfonaten.

6. Flüssige Reinigungsmittelzusammensetzung nach Anspruch 1, bei der das nichtionisches Tensid in einer Menge von 16 bis 22 Gew.-% vorhanden ist, das anionische Reinigungmittel in einer Menge von 2 bis 9 Gew.-% vorhanden ist und das Betain in einer Menge von 2 bis 9 Gew.-% vorhanden ist.

7. Flüssige Reinigungsmittelzusammensetzung nach Anspruch 6, bei der das anionische Reinigungsmittel C₁₂- bis C₁₆-Alkylsulfat ist.

8. Flüssige Reinigungsmittelzusammensetzung nach Anspruch 1, die ferner Konservierungsmittel enthält.

9. Flüssige Reinigungsmittelzusammensetzung nach Anspruch 1, die ferner Farbstabilisator enthält.

10. Verfahren zur Herstellung eines flüssigen Reinigigungsmittels gemäß Anspruch 2, bei dem zuerst das nichtionische Tensid mit dem Solubilisierungsmittel gemischt wird, dann unter Rühren das anionische Tensid und das Betain zugesetzt werden und schließlich unter Rühren die Formelmenge Wasser zugesetzt wird, um eine wäßrige Lösung der auf dem nichtionischen Tensid basierenden Tensidzusammensetzung zu bilden.

## Revendications

1. Un détergent liquide très moussant pour lavage doux à base d'agent tensio-actif non ionique, comprenant approximativement, en poids,
(a) 10 % à 30 % d'un agent tensio-actif non ionique hydrosoluble choisi dans le groupe formé par les produits de condensation d'alcanol primaire ou secondaire en C₈-C₁₈ avec 5 à 30 moles d'oxyde d'éthylène, les produits de condensation d'(alkyl en C₈-C₁₈)phenol avec 5 a 30 moles d'oxyde d'éthylène, les produits de condensation d'alcanol en C₈-C₂₀ avec un mélange hétérique d'oxyde d'éthylène et d'oxyde de propylène ayant un rapport en poids de l'oxyde d'éthylène à l'oxyde de propylène de 2,5:1 à 4:1 et une teneur totale en oxydes d'alkylène de 60 % à 85 % en poids, et les produits de condensation de 2 à 30 moles d'oxyde d'éthylène avec des mono- et triesters d'acides alcanoïques en C₁₀-C₂₀ du sorbitanne ayant un RHL de 8 à 15 ;
(b) 1 % à 10 % d'un détergent anionique hydrosoluble choisi dans le groupe formé par les alkylsulfates en C₈-C₁₈, les (alkyl en C₈-C₁₆)benzène-sulfonates, les paraffine-sulfonates en C₁₀-C₂₀, les alpha-oléfine-sulfonates en C₁₀-C₂₄ et les esters d'alkyle en C₈-C₁₈ de sulfosuccinates, les (acyl en C₈-C₁₈)iséthionates et les (acyl en C₈-C₁₈)-taurinates ; et
(c) 0,5 % à 10 % d'une bétaïne hydrosoluble ;
(d) le reste d'eau sous forme d'un milieu aqueux dans lequel ledit agent tensio-actif non ionique, ledit détergent anionique et ladite bétaïne sont solubilisés dans ladite eau, la somme de C et B étant de 15 % à 48 % en poids de la teneur totale en agents tensio-actifs de la composition, ledit agent tensio-actif non ionique représentant plus de 50 % en poids de la teneur totale en agents tensio-actifs, et ladite composition ne contenant pas de détergent anionique du type alkyl-polyoxyéthylène-sulfate, de détergent anionique consistant en laurylsulfate de triéthanolamine, d'amides, d'oxydes d'amine ni d'alcanolamides.

2. Une composition détergente liquide selon la revendication 1, qui contient, de plus, 1 % à 15 % en poids d'un agent solubilisateur choisi dans le groupe formé par les alcanols en C₂-C₃ mono- et dihydroxylés, les sels hydrosolubles d'hydrotropes du type benzènesulfonate à substitution en C₁-C₃, et leurs mélanges.

3. Une composition détergente liquide selon la revendication 2, dans laquelle de l'éthanol est présent en une proportion de 5 % en poids ou moins.

4. Une composition détergente liquide selon la revendication 2, dans laquelle ledit agent tensio-actif non ionique est ledit produit de condensation d'un alcanol primaire en C₈-C₁₈ avec 5 à 30 moles d'oxyde d'éthylène.

5. Une composition détergente liquide selon la revendication 4, dans laquelle ledit détergent anionique est choisi dans le groupe formé par les alkylsulfates en C₁₂-C₁₆, les (alkyl en C₁₀-C₁₅)benzène-sulfonates, les paraffine-sulfonates en C₁₃-C₁₇ et les alpha-oléfine-sulfonates en C₁₂-C₁₈.

6. Une composition détergente liquide selon la revendication 1, dans laquelle ledit agent tensio-actif non ionique est présent en une proportion de 16 % à 22 % en poids, ledit détergent anionique est présent en une proportion de 2 % à 9 % en poids et ladite bétaïne est présente en une proportion de 2 à 9 % en poids.

7. Une composition détergente liquide selon la revendication 6, dans laquelle ledit détergent anionique est un alkylsulfate en C₁₂-C₁₆.

8. Une composition détergente liquide selon la revendication 1, contenant de plus un conservateur.

9. Une composition détergente liquide selon la revendication 1, contenant de plus un stabilisant de couleur.

10. Un procédé de préparation du détergent liquide de la revendication 2, qui comprend les étapes consistant à mélanger d'abord ledit agent tensio-actif non ionique avec l'agent solubilisateur, ajouter successivement, sous agitation, ledit agent tensio-actif anionique et ladite bétaïne, et enfin ajouter, sous agitation, la quantité d'eau de la formule pour former une solution aqueuse de la composition à base d'agent tensio-actif non ionique.
